# EUROPEAN PATENT APPLICATION

(11) **EP 4 357 098 A1**
(43) Date of publication of application: **24.04.2024**
(21) Application number: 22848954.8
(22) Date of filing: 31.03.2022
(51) Int. Cl.: B29B 17/00, B01J 20/26, B01J 20/34

(54) **METHOD FOR PRODUCING SECONDARILY DEHYDRATED HIGHLY-WATER-ABSORBENT POLYMER FOR HIGHLY-WATER-ABSORBENT RECYCLED POLYMER, AND METHOD FOR PRODUCING HIGHLY-WATER-ABSORBENT RECYCLED POLYMER**

(30) Priority: 30.07.2021 JP 2021126208
(71) Applicant: Unicharm Corporation, Shikokuchuo-shi, Ehime 799-0111 (JP); SDP Global Co., Ltd., Tokyo, 105-0003 (JP)
(72) Inventor: KONISHI, Takayoshi, Kanonji-shi, Kagawa 769-1602 (JP); SUZUKI, Kazumitsu, Tokyo 103-0023 (JP); MORITA, Eiji, Tokyo 103-0023 (JP); HASEGAWA, Shimpei, Tokyo 103-0023 (JP)
(74) Representative: Dolleymores
(86) International application number: PCT/JP2022/016919
(87) International publication number: WO 2023/007871

(57) **Abstract**

The purpose of the present disclosure is to provide a method which is for producing a secondarily dehydrated highly-water-absorbent polymer for a highly-water-absorbent recycled polymer, and by which a highly-water-absorbent recycled polymer having a small residual amount of waste can be simply formed. The production method according to the present disclosure has the following feature.

A method for producing a secondarily dehydrated highly-water-absorbent polymer for a highly-water-absorbent recycled polymer is characterized by comprising: a preparation step for preparing a primarily dehydrated highly-water-absorbent polymer which is obtained by immersing a sanitary article-forming material, which forms a sanitary article, in an acid-containing aqueous solution that contains an acid to form the primarily dehydrated highly-water-absorbent polymer, and separating the primarily dehydrated highly-water-absorbent polymer from the sanitary article-forming material; and a secondary dehydration step for forming a secondarily dehydrated highly-water-absorbent polymer by bringing the primarily dehydrated highly-water-absorbent polymer into contact with a polyvalent metal ion supply source that can supply polyvalent metal ions to further dehydrate the primarily dehydrated highly-water-absorbent polymer.

## Description

### FIELD

The present disclosure relates to a method of manufacturing a secondarily dehydrated super absorbent polymer for a super absorbent recycled polymer, a method of manufacturing a super absorbent recycled polymer, and a method of manufacturing a super absorbent recycled polymer including pulp fibers and a super absorbent polymer having an acid group from a used sanitary product.

### BACKGROUND

The recycling of materials which configure a used sanitary product has been investigated.

For example, Patent Literature 1 discloses a method of regenerating a super absorbent polymer inactivated by an acid into a super absorbent recycled polymer having a predetermined water absorption property, including a preparation step of preparing a super absorbent polymer having an acid group and inactivated by an acid, a super absorbent recycled polymer formation step of forming the super absorbent recycled polymer in a wet state from the super absorbent polymer inactivated by the acid by adding an alkali metal ion supply source capable of supplying an alkali metal ion to an aqueous solution for regeneration containing the super absorbent polymer inactivated by the acid, and a drying step of drying the super absorbent recycled polymer in the wet state to form the super absorbent recycled polymer having a predetermined water absorption property (Claim 1).

In addition, Patent Literature 2 discloses a method of treating a water-absorbing resin including adding and mixing a weak acid alkali metal salt to an aggregate of a water-absorbing resin powder formed by water absorption of the water-absorbing resin powder (Claim 1), in which the aggregate of the water-absorbing resin powder is dehydrated with a divalent metal salt (Claim 2).

### [CITATION LIST]

### [PATENT LITERATURE]

Patent Literature 1: Japanese Unexamined Patent Publication No. 2019-135046
Patent Literature 2: Japanese Unexamined Patent Publication No. 2015-004034

### SUMMARY

### [TECHNICAL PROBLEM]

In the method described in Patent Literature 1, since the super absorbent polymer is inactivated (dehydrated) with an acid, ash is less likely to remain in the recycled pulp fibers. However, the dehydration degree of the super absorbent polymer is easily lowered, there is a possibility that excrement easily remains, and the drying energy when forming a super absorbent recycled polymer tends to increase.
In addition, in the method described in Patent Literature 2, while the dehydration degree of the super absorbent polymer is increased, the separation between the pulp fibers and the super absorbent polymer easily becomes insufficient, and the pulp fibers tend to remain in the super absorbent polymer. Further, in a case of forming recycled pulp fibers, ash tends to remain in the recycled pulp fibers.

Accordingly, the object of the present disclosure is to provide a method of manufacturing a secondarily dehydrated super absorbent polymer for a super absorbent recycled polymer, capable of easily forming a super absorbent recycled polymer with a small amount of remaining excrement.

### [SOLUTION TO PROBLEM]

The present inventors have found a method of manufacturing a secondarily dehydrated super absorbent polymer for a super absorbent recycled polymer, the method comprising: a preparation step of preparing a primarily dehydrated super absorbent polymer which is obtained by immersing a sanitary product configurational material which configures a sanitary product including pulp fibers and a super absorbent polymer having an acid group in an acid-containing aqueous solution including an acid to form the primarily dehydrated super absorbent polymer, and separating the primarily dehydrated super absorbent polymer from the sanitary product configurational material; and a secondary dehydration step of forming the secondarily dehydrated super absorbent polymer by bringing the primarily dehydrated super absorbent polymer into contact with a polyvalent metal ion supply source capable of supplying a polyvalent metal ion to further dehydrate the primarily dehydrated super absorbent polymer.

### [ADVANTAGEOUS EFFECT OF THE INVENTION]

The method of manufacturing a secondarily dehydrated super absorbent polymer for a super absorbent recycled polymer according to the present disclosure can easily form a super absorbent recycled polymer with a small amount of remaining excrement.

### DESCRIPTION OF EMBODIMENTS

Specifically, the present disclosure relates to the following aspects.

### [Aspect 1]

A method of manufacturing a secondarily dehydrated super absorbent polymer for a super absorbent recycled polymer, the method comprising:
a preparation step of preparing a primarily dehydrated super absorbent polymer which is obtained by immersing a sanitary product configurational material which configures a sanitary product including pulp fibers and a super absorbent polymer having an acid group in an acid-containing aqueous solution including an acid to form the primarily dehydrated super absorbent polymer, and separating the primarily dehydrated super absorbent polymer from the sanitary product configurational material; and
a secondary dehydration step of forming the secondarily dehydrated super absorbent polymer by bringing the primarily dehydrated super absorbent polymer into contact with a polyvalent metal ion supply source capable of supplying a polyvalent metal ion to further dehydrate the primarily dehydrated super absorbent polymer.

In the above-mentioned manufacturing method, in order to produce a super absorbent recycled polymer, a super absorbent polymer primarily dehydrated by an acid is prepared, and then, the primarily dehydrated super absorbent polymer is further dehydrated using a polyvalent metal ion supply source to form a secondarily dehydrated super absorbent polymer. In the secondarily dehydrated super absorbent polymer, the moisture content is low (the dehydration rate is high), that is, the amount of remaining excrement is small, and consequently, the amount of excrement remaining in a super absorbent recycled polymer can be lowered in comparison with a case of performing dehydration only using an acid (same type and same amount of acid) or a case of performing dehydration using a polyvalent metal ion supply source (same type and same amount of polyvalent metal ion supply source).
Further, a super absorbent recycled polymer can be easily formed by bringing the secondarily dehydrated super absorbent polymer formed by the above-mentioned manufacturing method into contact with an alkali metal ion supply source or the like.
It should be noted that, in the present specification, the terms "primarily dehydrated super absorbent polymer" and "secondarily dehydrated super absorbent polymer" may be referred to as "primarily dehydrated super absorbent polymer" and "secondarily dehydrated super absorbent polymer", respectively.

### [Aspect 2]

The method according to Aspect 1, wherein
in the secondary dehydration step, the primarily dehydrated super absorbent polymer is further dehydrated to have a moisture content of 50.0 to 80.0 mass% to form the secondarily dehydrated super absorbent polymer.

In the above-mentioned manufacturing method, since the secondarily dehydrated super absorbent polymer is formed by further dehydrating the primarily dehydrated super absorbent polymer to a predetermined moisture content, the amount of excrement remaining in the secondarily dehydrated super absorbent polymer and consequently, the super absorbent recycled polymer can be reduced. Further, the super absorbent recycled polymer can be easily formed by bringing the secondarily dehydrated super absorbent polymer formed by the above-mentioned manufacturing method into contact with an alkali metal ion supply source or the like.

### [Aspect 3]

The method according to Aspect 1 or 2, wherein
the polyvalent metal ion supply source is a hydroxide of an alkaline earth metal salt or a transition metal, a salt of a hydroxide of an alkaline earth metal salt or a transition metal and an acid, or an oxide of an alkaline earth metal salt or a transition metal.

In the above-mentioned manufacturing method, since the polyvalent metal ion supply source is selected from a predetermined substance, the amount of excrement remaining in the secondarily dehydrated super absorbent polymer and consequently, the super absorbent recycled polymer can be reduced. Further, the super absorbent recycled polymer can be easily formed by bringing the secondarily dehydrated super absorbent polymer formed by the above-mentioned manufacturing method into contact with an alkali metal ion supply source or the like.

### [Aspect 4]

The method according to any one of Aspects 1 to 3, wherein
in the secondary dehydration step, the secondarily dehydrated super absorbent polymer is formed by bringing the primarily dehydrated super absorbent polymer into contact with the polyvalent metal ion supply source or an aqueous solution of the polyvalent metal ion supply source.

In the above-mentioned manufacturing method, since the primarily dehydrated super absorbent polymer is brought into contact with the polyvalent metal ion supply source itself or an aqueous solution thereof, the amount of excrement remaining in the secondarily dehydrated super absorbent polymer and consequently, the super absorbent recycled polymer can be reduced. Further, the super absorbent recycled polymer can be easily formed by bringing the secondarily dehydrated super absorbent polymer formed by the above-mentioned manufacturing method into contact with an alkali metal ion supply source or the like.

### [Aspect 5]

The method according to Aspect 4, wherein
the secondary dehydration step further includes a drying step of drying the secondarily dehydrated super absorbent polymer.
In the above-mentioned manufacturing method, since the secondary dehydration step further includes a predetermined drying step, the moisture content of the secondarily dehydrated super absorbent polymer can be further lowered, and the amount of excrement remaining in the secondarily dehydrated super absorbent polymer and consequently, the super absorbent recycled polymer can be further reduced. Further, when the secondarily dehydrated super absorbent polymer formed by the above-mentioned manufacturing method can be brought into contact with an alkali metal ion supply source to form a super absorbent recycled polymer, the amount of the alkali metal ion supply source to be used can be reduced.

### [Aspect 6]

The method according to any one of Aspects 1 to 5, wherein
in the preparation step, the primarily dehydrated super absorbent polymer has a moisture content of more than 80.0 mass% and 97.0 mass% or less.

In the above-mentioned manufacturing method, in the preparation step, since the primarily dehydrated super absorbent polymer has a predetermined moisture content, the moisture content of the secondarily dehydrated super absorbent polymer is easily lowered (the amount of remaining excrement is easily lowered), and consequently, the amount of excrement remaining in the super absorbent recycled polymer can be reduced. Further, the super absorbent recycled polymer can be easily formed by bringing the secondarily dehydrated super absorbent polymer formed by the above-mentioned manufacturing method into contact with an alkali metal ion supply source or the like.

### [Aspect 7]

The method according to any one of Aspects 1 to 6, further comprising:
a primary dehydration step of immersing the sanitary product configurational material in the acid-containing aqueous solution to form the primarily dehydrated super absorbent polymer and separating the primarily dehydrated super absorbent polymer from the sanitary product configurational material, before the preparation step.

Since the above-mentioned manufacturing method further includes a predetermined primary dehydration step, the amount of excrement remaining in the secondarily dehydrated super absorbent polymer and consequently, the super absorbent recycled polymer can be reduced. Further, the super absorbent recycled polymer can be easily formed by bringing the secondarily dehydrated super absorbent polymer formed by the above-mentioned manufacturing method into contact with an alkali metal ion supply source or the like.

### [Aspect 8]

The method according to any one of Aspects 1 to 7, further comprising:
a super absorbent recycled polymer formation step of forming the super absorbent recycled polymer from the secondarily dehydrated super absorbent polymer by mixing the secondarily dehydrated super absorbent polymer and an alkali metal ion supply source capable of supplying an alkali metal ion in an aqueous solution for regeneration, after the secondary dehydration step.

The above-mentioned manufacturing method further includes a predetermined super absorbent recycled polymer formation step, and a super absorbent recycled polymer with a small amount of remaining excrement can be easily formed.

### [Aspect 9]

The method according to Aspect 8, wherein
the alkali metal ion supply source is (i) a hydroxide of an alkali metal having a standard electrode potential difference of 2.0 or less with the polyvalent metal ion, (ii) a salt of a hydroxide of an alkali metal having a standard electrode potential difference of 2.0 or less with the polyvalent metal ion and an acid which has an acid dissociation constant larger than an acid dissociation constant of the acid group of the super absorbent polymer, or (iii) an oxide of an alkali metal having a standard electrode potential difference of 2.0 or less with the polyvalent metal ion.

In the above-mentioned manufacturing method, since the alkali metal ion supply source is selected from predetermined sources, the polyvalent metal ions crosslinking the acid groups of the secondarily dehydrated super absorbent polymer are easily replaced with alkali metal ions, and consequently, the super absorbent recycled polymer has an excellent water absorption property. Due to that, a super absorbent recycled polymer with an excellent water absorption property and a small amount of remaining excrement can be easily formed.

### [Aspect 10]

The method according to Aspect 9, wherein
the alkali metal ion is selected from a group consisting of a lithium ion, a sodium ion, a potassium ion, and any combination of the lithium ion, the sodium ion, and the potassium ion.

In the above-mentioned manufacturing method, since the alkali metal ion is selected from predetermined ions, a super absorbent recycled polymer with an excellent water absorption property and a small amount of remaining excrement can be easily formed.

### [Aspect 11]

A method of manufacturing a super absorbent recycled polymer, comprising:
a preparation step of preparing a secondarily dehydrated super absorbent polymer which is obtained by immersing a sanitary product configurational material which configures a sanitary product including pulp fibers and a super absorbent polymer having an acid group in an acid-containing aqueous solution including an acid to form a primarily dehydrated super absorbent polymer, separating the primarily dehydrated super absorbent polymer from the sanitary product configurational material, and bringing the primarily dehydrated super absorbent polymer into contact with a polyvalent metal ion supply source capable of supplying a polyvalent metal ion to further dehydrate the primarily dehydrated super absorbent polymer; and
a super absorbent recycled polymer formation step of forming the super absorbent recycled polymer from the secondarily dehydrated super absorbent polymer by mixing the secondarily dehydrated super absorbent polymer and an alkali metal ion supply source capable of supplying an alkali metal ion in an aqueous solution for regeneration.

With the above-mentioned manufacturing method, a super absorbent recycled polymer with a small amount of remaining excrement can be easily formed.

### [Aspect 12]

A method of manufacturing a super absorbent recycled polymer from a used sanitary product including pulp fibers and a super absorbent polymer having an acid group, the method comprising:
a primary dehydration step of immersing a sanitary product configurational material which configures the sanitary product including the pulp fibers and the super absorbent polymer in an acid-containing aqueous solution including an acid to form a primarily dehydrated super absorbent polymer, and separating the primarily dehydrated super absorbent polymer from the sanitary product configurational material;
a secondary dehydration step of forming a secondarily dehydrated super absorbent polymer by bringing the primarily dehydrated super absorbent polymer into contact with a polyvalent metal ion supply source capable of supplying a polyvalent metal ion to further dehydrate the primarily dehydrated super absorbent polymer; and
a super absorbent recycled polymer formation step of forming the super absorbent recycled polymer from the secondarily dehydrated super absorbent polymer by mixing the secondarily dehydrated super absorbent polymer and an alkali metal ion supply source capable of supplying an alkali metal ion in an aqueous solution for regeneration.

With the above-mentioned manufacturing method, a super absorbent recycled polymer with a small amount of remaining excrement can be easily formed.

Hereinafter, (i) the method of manufacturing a secondarily dehydrated super absorbent polymer for a super absorbent recycled polymer (hereinafter, sometimes referred to as "the manufacturing method of the secondarily dehydrated super absorbent polymer"), (ii) the method of manufacturing a super absorbent recycled polymer (hereinafter, sometimes referred to as "the manufacturing method of the super absorbent recycled polymer", and (iii) the method of manufacturing a super absorbent recycled polymer from a used sanitary product including pulp fibers and a super absorbent polymer having an acid group (hereinafter, sometimes referred to as "the manufacturing method of the super absorbent recycled polymer from a used sanitary product" will be described in detail.

It should be noted that, from the viewpoint of ease of description, the following methods will be described in the order of: (iii) the manufacturing method of the super absorbent recycled polymer from a used sanitary product; (i) the manufacturing method of the secondarily dehydrated super absorbent polymer; and (ii) the manufacturing method of the super absorbent recycled polymer. In addition, the above-mentioned three manufacturing methods may be collectively referred to as "the manufacturing method of the present disclosure".

### <<Manufacturing Method of Super Absorbent Recycled Polymer From Used Sanitary Product>>

The manufacturing method of the super absorbent recycled polymer from a used sanitary product according to the present disclosure includes the following steps.
- a primary dehydration step of immersing a sanitary product configurational material which configures the sanitary product including the pulp fibers and the super absorbent polymer in an acid-containing aqueous solution including an acid to form a primarily dehydrated super absorbent polymer, and separating the primarily dehydrated super absorbent polymer from the sanitary product configurational material (hereinafter, sometimes referred to as "the primary dehydration step"),
- a secondary dehydration step of forming the secondarily dehydrated super absorbent polymer by bringing the primarily dehydrated super absorbent polymer into contact with a polyvalent metal ion supply source capable of supplying a polyvalent metal ion to further dehydrate the primarily dehydrated super absorbent polymer. (hereinafter, sometimes referred to as "the secondary dehydration step"), and
- a super absorbent recycled polymer formation step of forming the super absorbent recycled polymer from the secondarily dehydrated super absorbent polymer by mixing the secondarily dehydrated super absorbent polymer and an alkali metal ion supply source capable of supplying an alkali metal ion in an aqueous solution for regeneration (hereinafter, sometimes referred to as "the super absorbent recycled polymer formation step").

### [Primary Dehydration Step]

In the primary dehydration step, the sanitary product configurational material including the pulp fibers and the super absorbent polymer having an acid group is immersed in an acid-containing aqueous solution including an acid to form a primarily dehydrated super absorbent polymer and the primarily dehydrated super absorbent polymer is separated from the sanitary product configurational material.

In the primary dehydration step, the acid group of the super absorbent polymer having an acid group changes from the state of a salt (for example, sodium salt) with a component (for example, sodium ion) in a bodily fluid to the state of the free acid, and thus the water absorption property of the super absorbent polymer is lowered. When the super absorbent polymer which has absorbed water is put into an acid-containing aqueous solution, the negatively charged hydrophilic group (for example, -COO⁻) is neutralized by positively charged hydrogen ions (H⁺) (for example, - COOH), whereby the ionic repulsion of the hydrophilic group is weakened, the absorption power is lowered, and the super absorbent polymer is dehydrated.

The above-mentioned acid is not particularly limited, and for example, there are provided an inorganic acid and an organic acid. When the super absorbent polymer is dehydrated (inactivated) using an acid, in comparison with a case where the super absorbent polymer is dehydrated (inactivated) by using a polyvalent metal ion supply source capable of supplying a polyvalent metal ion, for example, lime, calcium chloride, magnesium sulfate, magnesium chloride, aluminum sulfate, aluminum chloride, and the like, ash is less likely to remain in the pulp fibers.

As the above-mentioned inorganic acid, for example, there are provided sulfuric acid, hydrochloric acid, and nitric acid, and the above-mentioned inorganic acid is preferably sulfuric acid from the viewpoint of not containing chlorine, of cost, and the like. As the above-mentioned organic acid, there are provided those having an acid group, for example, a carboxyl group, a sulfo group, and the like. It should be noted that an organic acid having a sulfo group is referred to as a sulfonic acid, and an organic acid having a carboxyl group and not having a sulfo group is referred to as a carboxylic acid. The above-mentioned organic acid is preferably an organic acid having a carboxyl group, particularly, a carboxylic acid, from the viewpoint of protecting the equipment.

In a case where the above-mentioned organic acid has a carboxyl group, the organic acid may have one or more carboxyl groups per one molecule, and preferably has a plurality of carboxyl groups per one molecule. Accordingly, it is easier for the organic acid to form chelate complexes with divalent or higher valent metals included in excrement, and the like, such as calcium, and in a case where recycled pulp fibers are formed from a used sanitary product, it is easier to lower the ash of the recycled pulp fibers to be formed.

Examples of the organic acid include citric acid, tartaric acid, malic acid, succinic acid, oxalic acid (the aforementioned being carboxylic acids with a plurality of carboxyl groups), gluconic acid (C6), pentanoic acid (C5), butanoic acid (C4), propionic acid (C3), glycolic acid (C2), acetic acid (C2), for example, glacial acetic acid, formic acid (C1) (the aforementioned being carboxylic acids with one carboxyl group), methanesulfonic acid, trifluoromethanesulfonic acid, benzenesulfonic acid, and p-toluenesulfone acid (the aforementioned being sulfonic acids).

It is preferable that the above-mentioned acid-containing aqueous solution has a predetermined pH, and the predetermined pH is preferably 4.5 or less, more preferably 4.0 or less, even more preferably 3.5 or less, and still even more preferably 3.0 or less. When the predetermined pH is too high, the primary dehydration of the super absorbent polymer is not sufficiently performed, and the primarily dehydrated super absorbent polymer tends to be less likely to be separated from the pulp fibers.

Further, the predetermined pH is preferably 0.5 or more, and more preferably 1.0 or more. When the predetermined pH is too low, there is a risk that the recycled pulp fibers may be damaged in a case where the recycled pulp fibers are formed from a used sanitary product.
It should be noted that, in the present specification, the pH means a value at 25°C. In addition, the pH can be measured using, for example, a twin pH meter AS-711 manufactured by HORIBA Co., Ltd.

In the manufacturing method of the present disclosure, it is preferable that the acid-containing aqueous solution satisfies the above predetermined pH at least at the time of the initiation of the primary dehydration step, for example, when the sanitary product configurational material is immersed in the acid-containing aqueous solution. This is for the purpose of performing the primary dehydration of the super absorbent polymer, and in a case where the primary dehydration of the super absorbent polymer is not sufficient, the discharge of the liquid such as bodily fluid, and the like retained by the super absorbent polymer tends to become insufficient, and further, the primarily dehydrated super absorbent polymer tends to be less likely to be separated from the pulp fibers and the like.
In the manufacturing method of the present disclosure, it is preferable that the predetermined pH is satisfied at the time of the termination of the primary dehydration step. This is from the viewpoint of maintaining a state where the super absorbent polymer is primarily dehydrated.

The super absorbent polymer is not particularly limited as long as it is used as a super absorbent polymer having an acid group in the technical field. For example, there are provided those including a carboxyl group, a sulfo group, and the like, and those including a carboxyl group are preferable.
As the super absorbent polymer including a carboxyl group, for example, there are provided those of polyacrylate based and polymaleic anhydride based, and as the super absorbent polymer including a sulfo group, there are provided those of polysulfonate based.
The above-mentioned pulp fibers are not particularly limited as long as the fibers can be included in sanitary products.

It should be noted that it is preferable that the acid for the primary dehydration of the super absorbent polymer has an acid dissociation constant (pKₐ, in water) smaller than the acid dissociation constant (pKₐ, in water) of the acid group included in the super absorbent polymer so that the super absorbent polymer is efficiently primarily dehydrated.

In a case where the above-mentioned acid has a plurality of acid groups, for example, in a case where the above-mentioned acid is a dibasic acid or a tribasic acid, it is preferable that the largest acid dissociation constant (pKₐ, in water) among the acid dissociation constants (pKₐ, in water) of the above-mentioned acid is smaller than the acid dissociation constant (pKₐ, in water) of the acid group of the super absorbent polymer, and, in a case where the super absorbent polymer has a plurality of types of acid groups, it is preferable that the largest acid dissociation constant (pKₐ, in water) among the acid dissociation constants (pKₐ, in water) of the above-mentioned acid is smaller than the smallest acid dissociation constant (pKₐ, in water) among the plurality of types of the acid groups of the super absorbent polymer. This is from the viewpoint of the primary dehydration efficiency of the super absorbent polymer.

In the present specification, as the acid dissociation constant (pKₐ, in water), the value described in Handbook of Electrochemistry edited by The Electrochemical Society of Japan can be adopted. According to the Handbook of Electrochemistry, the acid dissociation constants (pKₐ, in water, 25°C) of the major compounds are as follows.

### [Organic Acid]

- Tartaric acid: 2.99 (pKₐ₁), 4.44 (pKₐ₂)
- Malic acid: 3.24 (pKₐ₁), 4.71 (pKₐ₂)
- Citric acid: 2.87 (pKₐ₁), 4.35 (pKₐ₂), 5.69 (pKₐ₃)

### [Inorganic Acid]

### - Sulfuric acid: 1.99 (pKₐ₂)

The acid dissociation constant (pKₐ, in water) of the acids which is not described in the Handbook of Electrochemistry can be obtained by measurement. Examples of the device capable of measuring the acid dissociation constant (pKₐ, in water) include a compound physical property evaluation and analysis system, T3, manufactured by Sirius Analytical Instruments Ltd.

In the manufacturing method of the present disclosure, a specific method is not particularly limited as long as the sanitary product configurational material can be immersed in an acid-containing aqueous solution, and for example, the sanitary product configurational material may be thrown into a tank which contains the acid-containing aqueous solution, or the acid-containing aqueous solution may be poured into a tank in which the sanitary product configurational material is arranged.

The above-mentioned sanitary product is not particularly limited as long as the sanitary product includes pulp fibers and a super absorbent polymer, and for example, there are provided a disposable diaper, a disposable underpants, a sanitary napkin, a panty liner, a urine collection pad, a bed sheet, a pet sheet, and the like.
As the above-mentioned sanitary product, for example, there are provided those including a liquid-permeable sheet, a liquid-impermeable sheet, and an absorbent body (an absorbent core and a core wrap) interposed therebetween.

In the manufacturing method of the present disclosure, the sanitary product configurational material in the primary dehydration step can be a mixture of pulp fibers and a super absorbent polymer, for example, an absorbent core which is extracted from a used sanitary product. Further, the sanitary product configurational material may be the sanitary product itself.

In a case where the sanitary product configurational material to be immersed in the acid-containing aqueous solution includes, in addition to the pulp fibers and the super absorbent polymer (hereinafter, sometimes referred to as a "specific material"), an additional material (hereinafter, sometimes referred to as a "non-specific material"), for example, a liquid-permeable sheet, a liquid-impermeable sheet, or the like, the sanitary product itself may be immersed in the acid-containing aqueous solution, for example, as a sanitary product configurational material.

In the primary dehydration step, for example, the sanitary product configurational material is stirred for about 5 to 60 minutes, although depending on the temperature, in a primary dehydration tank containing an acid-containing aqueous solution, whereby the super absorbent polymer can be primarily dehydrated and the primarily dehydrated super absorbent polymer can be formed.

The acid-containing aqueous solution in the primary dehydration step may be heated or may not be heated.

In a case where the acid-containing aqueous solution is not heated, the temperature of the acid-containing aqueous solution in the primary dehydration step can be, for example, room temperature (25°C) to 40°C, lower than 60°C, or the like.

Specifically, in a case where the acid-containing aqueous solution is heated, the temperature of the acid-containing aqueous solution in the primary dehydration step is preferably higher than room temperature, more preferably, 60°C to 100°C, even more preferably 70°C to 95°C, and still even more preferably 80°C to 90°C. Accordingly, the acid contained in the acid-containing aqueous solution facilitates sterilization of bacteria derived from excrement and the like included in the acid-containing aqueous solution.

In the primary dehydration step, the super absorbent polymer is primarily dehydrated so that the primarily dehydrated super absorbent polymer has a moisture content of preferably more than 80.0 mass% and 97.0 mass% or less, more preferably 85.0 to 96.0 mass%, and even more preferably 87.0 to 95.0 mass%. Therefore, the moisture content of the secondarily dehydrated super absorbent polymer is easily lowered.

In the present specification, the moisture content is measured by using an infrared moisture meter FD-720 manufactured by Ketto Corporation. Specifically, approximately 5 g of a sample is placed on a sample dish of FD-720, a set temperature is set to 150°C, an automatic stop mode is selected, and the moisture content is measured.

### <Secondary Dehydration Step>

In the secondary dehydration step, a secondarily dehydrated super absorbent polymer is formed by bringing the primarily dehydrated super absorbent polymer into contact with a polyvalent metal ion supply source capable of supplying a polyvalent metal ion to further dehydrate the primarily dehydrated super absorbent polymer.

In the secondary dehydration step, the acid groups (for example, -COOHs) of the super absorbent polymer having acid groups are crosslinked with polyvalent metal ions (for example, calcium ions), and the primarily dehydrated super absorbent polymer is further dehydrated to form the secondarily dehydrated super absorbent polymer.

As the above-mentioned polyvalent metal ions, there are provided alkaline earth metal ions and transition metal ions.
As the above-mentioned alkaline earth metal ions, there are provided beryllium ions, magnesium ions, calcium ions, strontium ions, and barium ions. As the above-mentioned transition metal ions, there are provided iron ions, cobalt ions, nickel ions, and copper ions.

In a case where the above-mentioned polyvalent metal ion is an alkaline earth metal ion, as the polyvalent metal ion supply source capable of supplying the above-mentioned polyvalent metal ion, there are provided a hydroxide of an alkaline earth metal (for example, calcium hydroxide, or magnesium hydroxide), a hydroxide and an acid salt of an alkaline earth metal (for example, calcium chloride, calcium nitrate, magnesium chloride, or magnesium nitrate), an oxide of an alkaline earth metal (for example, calcium oxide, or magnesium oxide), and the like, and calcium chloride is preferable. As the above-mentioned acid, there are provided the acids for the acid-containing aqueous solution described in the section of "Primary Dehydration Step".

In a case where the above-mentioned polyvalent metal ion is a transition metal ion, as the polyvalent metal ion supply source capable of supplying the above-mentioned polyvalent metal ion, there are provided a hydroxide of a transition metal (for example, iron hydroxide, cobalt hydroxide, nickel hydroxide, or copper hydroxide), a hydroxide and an acid salt of a transition metal, an oxide of a transition metal (for example, iron oxide, cobalt oxide, nickel oxide, or copper oxide), and the like. As the above-mentioned acid, there are provided the acids for the acid-containing aqueous solution described in the section of "Primary Dehydration Step".

As specific example of the hydroxide and the acid salt of the transition metal, there is provided an inorganic acid salt or an organic acid salt. As the inorganic acid salt, for example, there are provided iron salts such as iron chloride, iron sulfate, iron phosphate, and iron nitrate, cobalt salts such as cobalt chloride, cobalt sulfate, cobalt phosphate, and cobalt nitrate, nickel salts such as nickel chloride and nickel sulfate, copper salts such as copper chloride and copper sulfate, and the like. As the organic acid salt, for example, there are provided iron lactate, cobalt acetate, cobalt stearate, nickel acetate, and copper acetate.

In the secondary dehydration step, for example, the secondarily dehydrated super absorbent polymer can be formed by bringing the primarily dehydrated super absorbent polymer into direct contact with the polyvalent metal ion supply source itself capable of supplying the polyvalent metal ion. In addition, in the secondary dehydration step, the primarily dehydrated super absorbent polymer is stirred for about 5 to 60 minutes, although depending on the temperature, in a secondary dehydration tank containing a polyvalent metal ion supply source-containing aqueous solution including the polyvalent metal ion supply source capable of supplying the polyvalent metal ion, whereby the primarily dehydrated super absorbent polymer can be secondarily dehydrated and the secondarily dehydrated super absorbent polymer can be formed.

The polyvalent metal ion supply source-containing aqueous solution in the secondary dehydration step may be heated or may not be heated.
In a case where the polyvalent metal ion supply source-containing aqueous solution is not heated, although not particularly limited, the temperature of the polyvalent metal ion supply source-containing aqueous solution in the secondary dehydration step can be, for example, room temperature (25°C) to 40°C, lower than 60°C, or the like.

In a case where the polyvalent metal ion supply source-containing aqueous solution is heated, the temperature of the polyvalent metal ion supply source-containing aqueous solution in the secondary dehydration step is preferably higher than room temperature, more preferably, 60°C to 100°C, even more preferably 70°C to 95°C, and still even more preferably 80°C to 90°C. In a case where the solubility of the polyvalent metal ion supply source in water is low, heating makes it possible to efficiently supply the polyvalent metal ion from the polyvalent metal ion supply source and to promote the formation of the secondarily dehydrated super absorbent polymer.

In the secondary dehydration step, the primarily dehydrated super absorbent polymer is secondarily dehydrated so that the secondarily dehydrated super absorbent polymer has a moisture content of preferably 50 to 80 mass%, more preferably 50 to 75 mass%, and even more preferably 50 to 67 mass%. Therefore, the amount of excrement remaining in the secondarily dehydrated super absorbent polymer can be reduced, and a super absorbent recycled polymer is easily formed from the secondarily dehydrated super absorbent polymer. It should be noted that, when the moisture content of the secondarily dehydrated super absorbent polymer is excessively lowered, the secondarily dehydrated super absorbent polymer is less likely to react with the alkali metal ion supply source in the next super absorbent recycled polymer formation step, and the super absorbent recycled polymer tends to be less likely to be formed.

In the secondary dehydration step, in a case where the primarily dehydrated super absorbent polymer is brought into contact with the polyvalent metal ion supply source-containing aqueous solution including the polyvalent metal ion supply source, for example, in a case where the primarily dehydrated super absorbent polymer is poured into the polyvalent metal ion supply source-containing aqueous solution, the concentration of the polyvalent metal ion supply source in the polyvalent metal ion supply source-containing aqueous solution is preferably 1.0 to 30.0 mass%, more preferably 3.0 to 25.0 mass%, and even preferably 5.0 to 20.0 mass%. This makes it easier for the secondarily dehydrated super absorbent polymer to have a desired moisture content.

### <Additional Drying Step>

In the manufacturing method of the present disclosure, the secondary dehydration step can further include a drying step of forming a dried secondarily dehydrated super absorbent polymer by drying the secondarily dehydrated super absorbent polymer after forming the secondarily dehydrated super absorbent polymer.

In the drying step, it is preferable that the secondarily dehydrated super absorbent polymer is dried so that the dried secondarily dehydrated super absorbent polymer has a moisture content of preferably 10 to 30 mass%, and more preferably 15 to 25 mass%. Therefore, the amount of the alkali metal ion supply source used when forming the super absorbent recycled polymer can be reduced and the water absorption property of the super absorbent recycled polymer to be formed can be maintained.

In the drying step, the secondarily dehydrated super absorbent polymer is dried at a drying temperature of preferably room temperature (for example, 25°C.) to 120°C, more preferably 30°C to 80°C, and even preferably 40°C to 60°C. When the drying temperature becomes low, the drying time tends to become long, and when the drying temperature becomes high, the acid groups of the super absorbent recycled polymer after drying cause dehydration condensation or the like. Due to that, there are cases where the water absorption property of the super absorbent recycled polymer after drying is lowered.

Further, the drying step can be performed, for example, for 30 to 300 minutes.

### <Super Absorbent Recycled Polymer Formation Step>

In the super absorbent recycled polymer formation step, the super absorbent recycled polymer is formed from the secondarily dehydrated super absorbent polymer by mixing the secondarily dehydrated super absorbent polymer and an alkali metal ion supply source capable of supplying an alkali metal ion in an aqueous solution for regeneration.

It should be noted that, in the present specification, the super absorbent polymer obtained by recycling the secondarily dehydrated super absorbent polymer may be referred to as the "super absorbent recycled polymer".

Although the alkali metal ion supply source is not particularly limited as long as the alkali metal ion supply source is capable of supplying an alkali metal ion, and for example, there are provided a hydroxide of an alkali metal, a salt of a hydroxide of an alkali metal and an acid (hereinafter, sometimes simply referred to as a "salt"), an oxides of an alkali metal, and the like.

As the alkali metal ion, there are provided a lithium ion, a sodium ion, a potassium ion, and any combination thereof. As the hydroxide of an alkali metal, there are provided lithium hydroxide, sodium hydroxide, potassium hydroxide, and any combinations thereof.

The salt can be an acid salt, a basic salt, or the like.

As the hydroxide of an alkali metal in the salt, there are provided lithium hydroxide, sodium hydroxide, potassium hydroxide, and any combinations thereof.

The acid in the salt is not particularly limited, and for example, there are provided the acids for the acid-containing aqueous solution, which are described in the section of "Primary Dehydration Step" (for example, hydrochloric acid, and sulfuric acid), carbonic acid, and the like.

As the oxide of an alkali metal, there are provided lithium oxide, sodium oxide, potassium oxide, and any combinations thereof.

The alkali metal ion supply source and the above-described polyvalent metal ion supply source have a relationship that the difference of the standard electrode potential between the alkali metal ion constituting the alkali metal ion supply source and the polyvalent metal ion constituting the polyvalent metal ion supply source is preferably 2.0 or less, more preferably 1.6 or less, further preferably 1.0 or less, and even more preferably 0.5 or less. Therefore, the polyvalent metal ions crosslinking the acid groups of the secondarily dehydrated super absorbent polymer are easily replaced with alkali metal ions, and consequently, the super absorbent recycled polymer has an excellent water absorption property.

It is preferable that the above-mentioned acid is an acid having an acid dissociation constant (pKₐ, in water) that is larger than the acid dissociation constant (pKₐ, in water) of the acid group in the super absorbent polymer. This makes it easier for the acid group of the super absorbent polymer to form an alkali metal salt.

In a case where the above-mentioned acid has a plurality of acid groups, for example, in a case where the above-mentioned acid is a dibasic acid or a tribasic acid, it is preferable that the smallest acid dissociation constant (pKₐ, in water) among the acid dissociation constants (pKₐ, in water) of the above-mentioned acid is larger than the acid dissociation constant (pKₐ, in water) of the acid group of the super absorbent polymer, and, in a case where the super absorbent polymer has a plurality of types of acid groups, it is preferable that the smallest acid dissociation constant (pKₐ, in water) among the acid dissociation constants (pKₐ, in water) of the above-mentioned acid is larger than the largest acid dissociation constant (pKₐ, in water) among the plurality of types of the acid groups of the super absorbent polymer. This is from the viewpoint of making it easier for the acid group of the super absorbent polymer to form an alkali metal salt.

The carbonic acid as the above-mentioned acid is preferable, since it is difficult for carbonic acid to remain in an aqueous solution for regeneration, or it is easy for carbonic acid to be removed by being heated, or the like.
Further, although depending on the pH of the super absorbent recycled polymer, for example, in a case where the super absorbent recycled polymer has an acidic pH, the above-mentioned acids other than carbonic acid can give an antibacterial property to the super absorbent recycled polymer.

As the above-mentioned salt, there are provided lithium carbonate, sodium carbonate, potassium carbonate, lithium hydrogen carbonate, sodium hydrogen carbonate, potassium hydrogen carbonate, lithium chloride, sodium chloride, potassium chloride, and the like.

In the super absorbent recycled polymer formation step, the aqueous solution for regeneration may be acidic (pH < 7.0), neutral (pH = 7.0) or alkaline (7.0 < pH), and the aqueous solution for regeneration is preferably neutral or alkaline, and is preferably alkaline. This is from the viewpoint of effects of the present disclosure.

In order to make the aqueous solution for regeneration acidic, neutral, or alkaline, the aqueous solution for regeneration may include an acidic substance, an alkaline substance, or a buffer substance (a buffer solution), and the above-mentioned alkali metal ion supply source may include an acidic substance, an alkaline substance, or a buffer substance (a buffer solution).
The above-mentioned acidic substance, an alkaline substance, or a buffer substance (a buffer solution) is preferable in a case where it is difficult for the alkali metal ion supply source to change the pH of the aqueous solution for regeneration (for example, in a case where the alkali metal ion supply source is lithium chloride, sodium chloride, potassium chloride, or the like), in a case where the pH of the aqueous solution for regeneration is not desired to be changed, and the like.

The above-mentioned acidic substance is not particularly limited as long as the acidic substance can make the pH of deionized water be acidic (pH < 7.0), those known in the technical field can be adopted, and for example, there are provided the acids for the acid-containing aqueous solution, which are described in the section of "Primary Dehydration Step", carbonic acid, and the like. The above-mentioned alkaline substance is not particularly limited as long as the alkaline substance can make the pH of deionized water be alkaline (7.0 < pH), those known in the technical field can be adopted, and for example, there are provided a hydroxide of an alkali metal or a salt thereof, a hydroxide of an alkaline earth metal or a salt thereof, and the like. From the viewpoint of making it difficult for the super absorbent polymer to be inactivated, a hydroxide of an alkali metal or a salt thereof is preferable. The hydroxide of an alkali metal or the salt thereof is as mentioned above.
As the above-mentioned buffer substance (a buffer solution), for example, there is provided a carbonic acid-bicarbonate buffer solution which includes sodium carbonate, sodium hydrogen carbonate, and water.

The above-mentioned aqueous solution for regeneration is not particularly limited as long as the aqueous solution includes water, and may be an aqueous solution which is substantially neutral (for example, tap water, deionized water, or the like), an acidic aqueous solution (an aqueous solution which satisfies pH < 7.0), for example, the acid-containing aqueous solution which is used in the primary dehydration step, the acid-containing aqueous solution including an inorganic acid or organic acid, which is described in the section of the "Primary Dehydration Step", or an alkaline aqueous solution (an aqueous solution which satisfies 7.0 < pH), for example, an aqueous solution including the above-mentioned alkali metal ion supply source.

By adjusting the pH of the aqueous solution for regeneration, the water absorption property of the super absorbent recycled polymer can be adjusted to a predetermined range.
The above-mentioned pH is not particularly limited as long as the super absorbent recycled polymer can achieve a predetermined water absorption property (absorption ratio), and may be any of a region of acidic pH (pH < 7.0), a neutral pH (pH = 7.0), and a region of alkaline pH (7.0 < pH), and the pH is preferably 5.0 to 14.0.

In a case where the pH is adjusted within the acidic pH region, the above-mentioned aqueous solution for regeneration is preferably an acidic aqueous solution, and it is preferable that an alkali metal ion supply source is added as it is or as an aqueous solution to an aqueous solution for regeneration, which is an acidic aqueous solution, to form the super absorbent recycled polymer.

In a case where the pH is adjusted within the neutral pH or alkaline pH region, the above-mentioned aqueous solution for regeneration may be an acidic aqueous solution, a substantially neutral aqueous solution, or an alkaline aqueous solution, and is preferably a substantially neutral aqueous solution, or an alkaline aqueous solution. This is from the viewpoint of efficiently using the alkali metal ion supply source for the formation of the super absorbent recycled polymer. Further, it is preferable that an alkali metal ion supply source is added as it is or as an aqueous solution to an aqueous solution for regeneration which is a substantially neutral aqueous solution or an alkaline aqueous solution to form the super absorbent recycled polymer.

The alkali metal ion supply source may be present in the aqueous solution for regeneration as mentioned above, and the alkali metal ion supply source can be added to the aqueous solution for regeneration as it is or as an aqueous solution.
In a case where a hydroxide of an alkali metal is added to an aqueous solution for regeneration as the alkali metal ion supply source, it is preferable that the hydroxide of an alkali metal is an aqueous solution, and the aqueous solution is added to the aqueous solution for regeneration with such a concentration that the hydroxide ion concentration is preferably 0.1 to 5.0 mol/L, more preferably 0.3 to 3.0 mol/L, and even more preferably 0.4 to 1.0 mol/L. This is from the viewpoint of ease of adjusting the pH of the aqueous solution for regeneration and consequently, the water absorption property of the super absorbent recycled polymer.

The super absorbent recycled polymer formation step can be performed by stirring or the like the aqueous solution for regeneration at a predetermined temperature, for example, 2°C to 80°C, for a predetermined time, for example, 5 to 60 minutes.

### <Additional Drying Step>

The manufacturing method of the present disclosure can further include a drying step after the super absorbent recycled polymer formation step.

In the drying step, the super absorbent recycled polymer formed in the super absorbent recycled polymer formation step (hereinafter, sometimes referred to as a "super absorbent recycled polymer before drying" for distinction) is dried to form a super absorbent recycled polymer having a predetermined water absorption property (hereinafter, sometimes referred to as a "super absorbent recycled polymer after drying" for distinction).

The predetermined water absorption property is not particularly limited, but considering the practicability of the super absorbent recycled polymer after drying, the water absorption ratio has any value of preferably 100 times (g/g) or more, more preferably 200 times (g/g) or more, and even more preferably 300 times (g/g) or more with respect to deionized water.

The predetermined water absorption property is not particularly limited, but considering the gel strength at the time when the super absorbent recycled polymer after drying is swollen, the water absorption ratio has any value of preferably 500 times (g/g) or less, more preferably 450 times (g/g) or less, and even more preferably 400 times (g/g) or less with respect to deionized water.

In the present specification, the water absorption ratio is measured as follows.
(1) A sample (super absorbent recycled polymer) is placed in a mesh and suspended for 5 minutes, the moisture attached to the surfaces of the sample is removed, and the mass thereof before drying: mi (g) is measured.
(2) The sample whose mass before drying is measured is dried at 120°C for 120 minutes, and the mass thereof after drying: m₂ (g) is measured.
(3) The water absorption ratio (g / g) is calculated by the following formula.

### Water absorption ratio (g / g) = m₁/m₂

In the drying step, the aqueous solution for regeneration including the super absorbent recycled polymer before drying, which is obtained in the super absorbent recycled polymer formation step, is directly dried, and thus the super absorbent recycled polymer after drying can be obtained. Further, from the aqueous solution for regeneration including the super absorbent recycled polymer, the super absorbent recycled polymer before drying is separated from the aqueous solution for regeneration by using a device which is capable of solid-liquid separation, and then the super absorbent recycled polymer after drying can be obtained.

The separated super absorbent recycled polymer before drying may be washed with tap water, deionized water, or the like before drying, and the aqueous solution for regeneration that is attached to the surface of the super absorbent recycled polymer before drying may be removed.
Further, the separated super absorbent recycled polymer before drying can be brought into contact with a hydrophilic organic solvent (for example, immersed in a hydrophilic organic solvent) before drying, whereby the moisture included in the separated super absorbent recycled polymer can be dehydrated to a water absorption ratio of preferably 100 times (g/g) or less, more preferably 70 times (g/g) or less, and even more preferably 50 times (g/g) or less. This makes it possible to lower the drying temperature and/or to reduce the drying time.

The above-mentioned hydrophilic organic solvent is preferably those that are miscible with water, and for example, there are provided an alcohol-based solvent (for example, methanol, ethanol, propyl alcohol, isomers thereof, butyl alcohol, and isomers thereof), a ketone-based solvent (for example, acetone or methyl ethyl ketone), a nitrile-based solvent (for example, acetonitrile), and the like.

In the drying step, the super absorbent recycled polymer is dried at a drying temperature of preferably room temperature (for example, 25°C) to 120°C, more preferably 30°C to 80°C, and even more preferably 40°C to 60°C. When the drying temperature becomes low, the drying time tends to become long, and when the drying temperature becomes high, the acid groups of the super absorbent recycled polymer after drying cause dehydration condensation or the like. Due to that, there are cases where the water absorption property of the super absorbent recycled polymer after drying is lowered.
In a case where the separated super absorbent recycled polymer before drying is brought into contact with a hydrophilic organic solvent before drying, the drying temperature can be lowered, for example, from room temperature to 60°C.

The drying step may be performed under reduced pressure, for example, at a pressure of 0.1 kPa or more and less than 100 kPa.

The drying step can be performed, for example, for 30 to 300 minutes.

It is preferable that the drying step is performed in a manner such that the loss on drying of the super absorbent recycled polymer is preferably 15% or less (2.0 g, 105°C, 3 hours). This is from the viewpoint of using the super absorbent recycled polymer after drying.

The above-described weight loss on drying is measured according to "7. Weight loss test method" of <2. General test method> of "Standards for materials of sanitary treatment products" which is attached as a separate sheet to "Regarding the standards for materials of sanitary treatment products" notified as the Pharmaceutical and food examination issue 0325 No. 24 on March 25, 2015 by The Ministry of health, labor and welfare.

After the drying step, in a case in which the dried super absorbent recycled polymer after drying is fixed, integrated, or the like, the crushing, classification, or the like, of the super absorbent recycled polymer after drying may be performed.

### <<Manufacturing Method of Secondarily Dehydrated Super Absorbent Polymer>>

The manufacturing method of the secondarily dehydrated super absorbent polymer according to the present disclosure includes the following steps.
- a preparation step of preparing a primarily dehydrated super absorbent polymer which is obtained by immersing a sanitary product configurational material which configures a sanitary product including pulp fibers and a super absorbent polymer having an acid group in an acid-containing aqueous solution including an acid to form the primarily dehydrated super absorbent polymer, and separating the primarily dehydrated super absorbent polymer from the sanitary product configurational material (hereinafter, sometimes referred to as "the preparation step"), and
- a secondary dehydration step of forming a secondarily dehydrated super absorbent polymer by bringing the primarily dehydrated super absorbent polymer into contact with a polyvalent metal ion supply source capable of supplying polyvalent metal ion to further dehydrate the primarily dehydrated super absorbent polymer (hereinafter, sometimes referred to as "the secondary dehydration step"), and

### <Preparation Step>

The primarily dehydrated super absorbent polymer in the preparation step can be prepared by, for example, performing the "Primary Dehydration Step" in the "Manufacturing Method of Super

Absorbent Recycled Polymer From Used Sanitary Product".

### <Secondary Dehydration Step>

The secondary dehydration step is the same as the "Secondary Dehydration Step" in the "Manufacturing Method of Super Absorbent Recycled Polymer From Used Sanitary Product" and therefore description thereof will be omitted.

### <<Manufacturing Method of Super Absorbent Recycled Polymer>>

The manufacturing method of the super absorbent recycled polymer according to the present disclosure includes the following steps.
- a preparation step of preparing a secondarily dehydrated super absorbent polymer which is obtained by immersing a sanitary product configurational material which configures a sanitary product including pulp fibers and a super absorbent polymer having an acid group in an acid-containing aqueous solution including an acid to form a primarily dehydrated super absorbent polymer, separating the primarily dehydrated super absorbent polymer from the sanitary product configurational material, and bringing the primarily dehydrated super absorbent polymer into contact with a polyvalent metal ion supply source capable of supplying a polyvalent metal ion to further dehydrate the primarily dehydrated super absorbent polymer (hereinafter, sometimes referred to as the "preparation step"), and
- a super absorbent recycled polymer formation step of forming the super absorbent recycled polymer from the secondarily dehydrated super absorbent polymer by mixing the secondarily dehydrated super absorbent polymer and an alkali metal ion supply source capable of supplying an alkali metal ion in an aqueous solution for regeneration (hereinafter, sometimes referred to as "the super absorbent recycled polymer formation step").

### <Preparation Step>

The secondarily dehydrated super absorbent polymer in the preparation step can be prepared by, for example, performing the "primary dehydration step" and the "secondary dehydration step" in the "Manufacturing Method of Super Absorbent Recycled Polymer From Used Sanitary Product".

### <Super Absorbent Recycled Polymer Formation Step>

The super absorbent recycled polymer formation step is the same as "the super absorbent recycled polymer formation step" in the "Manufacturing Method of Super Absorbent Recycled Polymer From Used Sanitary Product" and therefore description thereof will be omitted.

The super absorbent recycled polymer can be used without any particular limitation in applications in which a super absorbent polymer is used, and for example, may be used for a sanitary product (for example, a disposable diaper, an incontinence pad (for example, a light incontinence pad), a disposable underpants, a sanitary napkin, a panty liner, and a bed sheet), a pet sheet, cat sand, a soil conditioning agent, and the like.

### Examples

### [Example 1]

100 g of a polyacrylic acid-based super absorbent polymer (AQUA KEEP, manufactured by SUMITOMO SEIKA CHEMICALS CO., LTD., which was unused, hereinafter, referred to as "SAP") was allowed to absorb saline, and then filtered through a 200-mesh filter to prepare simulated used SAP No. 1 with a moisture content of 97.5 mass%.

Simulated used SAP No. 1 was stirred for 5 minutes in 4,000 mL of a 1.0 mass% citric acid aqueous solution as an acid-containing aqueous solution, left to stand for 20 minutes, and then filtered through a 200-mesh filter to form primarily dehydrated SAP No. 1. The moisture content of primarily dehydrated SAP No. 1 was 95.0 mass%.

Primarily dehydrated SAP No. 1 was stirred for 5 minutes in 4,000 mL of a 10.0 mass% calcium hydroxide dispersion solution as a polyvalent metal ion supply source, left to stand for 1 hour, filtered through a 200-mesh filter, and then dehydrated using a centrifugal dehydrator at 150G for 5 minutes to form secondarily dehydrated SAP No. 1. It should be noted that, although solid calcium hydroxide was also contained in the 10.0 mass% calcium hydroxide (Ca(OH)₂) dispersion solution, it was confirmed that the solid calcium hydroxide was removed by filtering through the 200-mesh filter and then dehydration using the centrifugal dehydrator. The moisture content of secondarily dehydrated SAP No. 1 was 69.0 mass%.

By drying secondarily dehydrated SAP No. 1 at 120°C for 120 minutes, secondarily dehydrated SAP No. 1 after drying was obtained.
The drying property of the secondarily dehydrated SAP after drying was determined based on the following criteria. The results are shown in Table 1.

### [Drying Property]

O: Drying of the secondarily dehydrated SAP after drying is sufficient, and the secondarily dehydrated SAP can be in the form of powder or can be easily made into the form of powder.

X: Drying of the secondarily dehydrated SAP after drying is insufficient and is in the form of lump.

### [Examples 2 to 4]

Primarily dehydrated SAP Nos. 2 to 4 and secondarily dehydrated SAP Nos. 2 to 4 were formed by changing the acid-containing aqueous solution and the polyvalent metal ion supply source as shown in Table 1 and the moisture contents thereof were measured. Further, secondarily dehydrated SAP Nos. 2 to 4 after drying were formed and the drying properties thereof were evaluated. The results are shown in Table 1.

### [Comparative Example 1]

Simulated used SAP No. 1 was stirred for 5 minutes in 4,000 mL of a 1.0 mass% sulfuric acid aqueous solution as an acid-containing aqueous solution, left to stand for 20 minutes, and filter through a 200-mesh filter to form primarily dehydrated SAP No. 5. The moisture content of primarily dehydrated SAP No. 5 was 90.0 mass%.

By drying primarily dehydrated SAP No. 5 at 120°C for 120 minutes, primarily dehydrated SAP No. 5 after drying was obtained and the drying property thereof was evaluated. The results are shown in Table 1.

### [Comparative Example 2]

Simulated used SAP No. 1 was stirred for 5 minutes in 4,000 mL of a 10.0 mass% calcium hydroxide dispersion solution as a polyvalent metal ion supply source, left to stand for 1 hour, filtered through a 200-mesh filter, and then dehydrated using a centrifugal dehydrator at 150G for 5 minutes to form primarily dehydrated SAP No. 6. The moisture content of primarily dehydrated SAP No. 6 was 73.0 mass%.

By drying primarily dehydrated SAP No. 6 at 120°C for 120 minutes, primarily dehydrated SAP No. 6 after drying was obtained and the drying property thereof was evaluated. The results are shown in Table 1.

### [Table 1]

**Table 1**

| | Moisture content/mass% | | | | | |
|---|---|---|---|---|---|---|
| | Example 1 | Example 2 | Example 3 | Example 4 | Comparative Example 1 | Comparative Example 2 |
| Simulated used SAP | | | | | | |
| No. | No. 1 | | | | | |
| | 97.5 | | | | | |

| Primarily dehydrated SAP | | | | | | |
|---|---|---|---|---|---|---|
| No. | No. 1 | No. 2 | No. 3 | No. 4 | No. 5 | - |
| 1.0 mass% citric acid aqueous solution | 95.0 | | | | | |
| 0.1 mass% sulfuric acid aqueous solution | | 940 | | | | |
| 1.0 mass% sulfuric acid aqueous solution | | | 90.0 | 90.0 | 90.0 | |

| Secondarily (primarily) dehydrated SAP | | | | | | |
|---|---|---|---|---|---|---|
| No. | No. 1 | No. 2 | No. 3 | No. 4 | - | No. 6 |
| 10.0 mass% Ca(OH)₂ dispersion solution | 69.0 | 67.0 | 62.0 | | | 73.0 |
| 100 mass% CaCl₂ aqueous solution | | | | 57.0 | | |

| Secondarily (primarily) dehydrated SAP after drying | | | | | | |
|---|---|---|---|---|---|---|
| No. | No. 1 | No. 2 | No. 3 | No. 4 | No. 5 | No. 6 |
| Drying property | O | O | O | O | X | X |

Secondarily dehydrated SAP Nos. 1 to 4 were immersed in a sodium hydroxide aqueous solution to form super absorbent recycled polymers. Then, primarily dehydrated SAP Nos. 5 and 6 were compared, and super absorbent recycled polymers could be formed using (i) a low concentration sodium hydroxide aqueous solution, and (ii) a small amount of a sodium hydroxide aqueous solution.

Further, secondarily dehydrated SAP Nos. 1 to 4 after drying were immersed in a sodium hydroxide aqueous solution to form super absorbent recycled polymers. Then, secondarily dehydrated SAP Nos. 1 to 4 were compared, and super absorbent recycled polymers could be formed using (i) a low concentration sodium hydroxide aqueous solution, and (ii) a small amount of a sodium hydroxide aqueous solution.

### Example 5

A mixture of 100 g of a polyacrylic acid-based super absorbent polymer (AQUA KEEP, manufactured by SUMITOMO SEIKA CHEMICALS CO., LTD., which was unused, hereinafter, referred to as "SAP") and 100 g of pulp fibers (softwood pulp fibers) was allowed to absorb 4,000 mL of saline. Then, the swollen SAP and the pulp fibers were integrated. The saline-absorbed mixture was filtered through a 200-mesh filter to remove the remaining saline from the mixture.

The mixture from which the saline had been removed was stirred in 4,000 mL of a 1.0 mass% citric acid aqueous solution as an acid-containing aqueous solution for 5 minutes, and allowed to stand for 20 minutes, and then the pulp fibers and the primarily dehydrated SAP were separated. The primarily dehydrated SAP remaining in the pulp fibers was decomposed with an ozone-containing gas according to an oxidant treatment step P19 of Patent Literature 1, and recycled pulp fibers No. 1 were obtained. The ash content of recycled pulp fibers No. 1 was 0.12 mass%.

### Example 6

Recycled pulp fibers No. 2 were obtained in the same manner as in Example 5 except that "4,000 mL of a 1.0 mass% citric acid aqueous solution" was changed to "4,000 mL of a 1.0 mass% sulfuric acid aqueous solution". The ash content of recycled pulp fibers No. 2 was 0.15 mass%.

### <Comparative Example 3>

Recycled pulp fibers No. 3 were obtained in the same manner as in Example 5 except that "4,000 mL of a 1.0 mass% citric acid aqueous solution" was changed to "4,000 mL of a 10.0 mass% calcium hydroxide dispersion solution". The ash content of recycled pulp fibers No. 3 was 12.1 mass%.

## Claims

1. A method of manufacturing a secondarily dehydrated super absorbent polymer for a super absorbent recycled polymer, the method comprising:
a preparation step of preparing a primarily dehydrated super absorbent polymer which is obtained by immersing a sanitary product configurational material which configures a sanitary product including pulp fibers and a super absorbent polymer having an acid group in an acid-containing aqueous solution including an acid to form the primarily dehydrated super absorbent polymer, and separating the primarily dehydrated super absorbent polymer from the sanitary product configurational material; and
a secondary dehydration step of forming the secondarily dehydrated super absorbent polymer by bringing the primarily dehydrated super absorbent polymer into contact with a polyvalent metal ion supply source capable of supplying a polyvalent metal ion to further dehydrate the primarily dehydrated super absorbent polymer.

2. The method according to Claim 1, wherein
in the secondary dehydration step, the primarily dehydrated super absorbent polymer is further dehydrated to have a moisture content of 50.0 to 80.0 mass% to form the secondarily dehydrated super absorbent polymer.

3. The method according to Claim 1 or 2, wherein
the polyvalent metal ion supply source is a hydroxide of an alkaline earth metal salt or a transition metal, a salt of a hydroxide of an alkaline earth metal salt or a transition metal and an acid, or an oxide of an alkaline earth metal salt or a transition metal.

4. The method according to any one of Claims 1 to 3, wherein
in the secondary dehydration step, the secondarily dehydrated super absorbent polymer is formed by bringing the primarily dehydrated super absorbent polymer into contact with the polyvalent metal ion supply source or an aqueous solution of the polyvalent metal ion supply source.

5. The method according to Claim 4, wherein
the secondary dehydration step further includes a drying step of drying the secondarily dehydrated super absorbent polymer.

6. The method according to any one of Claims 1 to 5, wherein
in the preparation step, the primarily dehydrated super absorbent polymer has a moisture content of more than 80.0 mass% and 97.0 mass% or less.

7. The method according to any one of Claims 1 to 6, further comprising:
a primary dehydration step of immersing the sanitary product configurational material in the acid-containing aqueous solution to form the primarily dehydrated super absorbent polymer and separating the primarily dehydrated super absorbent polymer from the sanitary product configurational material, before the preparation step.

8. The method according to any one of Claims 1 to 7, further comprising:
a super absorbent recycled polymer formation step of forming the super absorbent recycled polymer from the secondarily dehydrated super absorbent polymer by mixing the secondarily dehydrated super absorbent polymer and an alkali metal ion supply source capable of supplying an alkali metal ion in an aqueous solution for regeneration, after the secondary dehydration step.

9. The method according to Claim 8, wherein
the alkali metal ion supply source is (i) a hydroxide of an alkali metal having a standard electrode potential difference of 2.0 or less with the polyvalent metal ion, (ii) a salt of a hydroxide of an alkali metal having a standard electrode potential difference of 2.0 or less with the polyvalent metal ion and an acid which has an acid dissociation constant larger than an acid dissociation constant of the acid group of the super absorbent polymer, or (iii) an oxide of an alkali metal having a standard electrode potential difference of 2.0 or less with the polyvalent metal ion.

10. The method according to Claim 9, wherein
the alkali metal ion is selected from a group consisting of a lithium ion, a sodium ion, a potassium ion, and any combination of the lithium ion, the sodium ion, and the potassium ion.

11. A method of manufacturing a super absorbent recycled polymer, comprising:
a preparation step of preparing a secondarily dehydrated super absorbent polymer which is obtained by immersing a sanitary product configurational material which configures a sanitary product including pulp fibers and a super absorbent polymer having an acid group in an acid-containing aqueous solution including an acid to form a primarily dehydrated super absorbent polymer, separating the primarily dehydrated super absorbent polymer from the sanitary product configurational material, and bringing the primarily dehydrated super absorbent polymer into contact with a polyvalent metal ion supply source capable of supplying a polyvalent metal ion to further dehydrate the primarily dehydrated super absorbent polymer; and
a super absorbent recycled polymer formation step of forming the super absorbent recycled polymer from the secondarily dehydrated super absorbent polymer by mixing the secondarily dehydrated super absorbent polymer and an alkali metal ion supply source capable of supplying an alkali metal ion in an aqueous solution for regeneration.

12. A method of manufacturing a super absorbent recycled polymer from a used sanitary product including pulp fibers and a super absorbent polymer having an acid group, the method comprising:
a primary dehydration step of immersing a sanitary product configurational material which configures the sanitary product including the pulp fibers and the super absorbent polymer in an acid-containing aqueous solution including an acid to form a primarily dehydrated super absorbent polymer, and separating the primarily dehydrated super absorbent polymer from the sanitary product configurational material;
a secondary dehydration step of forming a secondarily dehydrated super absorbent polymer by bringing the primarily dehydrated super absorbent polymer into contact with a polyvalent metal ion supply source capable of supplying a polyvalent metal ion to further dehydrate the primarily dehydrated super absorbent polymer; and
a super absorbent recycled polymer formation step of forming the super absorbent recycled polymer from the secondarily dehydrated super absorbent polymer by mixing the secondarily dehydrated super absorbent polymer and an alkali metal ion supply source capable of supplying an alkali metal ion in an aqueous solution for regeneration.
